(19) Europäisches Patentamt / European Patent Office / Office européen des brevets

(11) **EP 3 281 647 A1**

(12) **EUROPEAN PATENT APPLICATION**
published in accordance with Art. 153(4) EPC

(43) Date of publication:
**14.02.2018 Bulletin 2018/07**

(51) Int Cl.:
**A61L 27/00** [(2006.01)]

(21) Application number: **16776562.7**

(22) Date of filing: **06.04.2016**

(86) International application number:
**PCT/JP2016/061260**

(87) International publication number:
**WO 2016/163396 (13.10.2016 Gazette 2016/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **08.04.2015 JP 2015078912**

(71) Applicant: **Toyobo Co., Ltd.
Osaka-shi
Osaka 530-8230 (JP)**

(72) Inventors:
• **IWAI, Atsushi
Otsu-shi
Shiga 520-0292 (JP)**
• **KAJII, Fumihiko
Otsu-shi
Shiga 520-0292 (JP)**
• **TANAKA, Hidenori
Otsu-shi
Shiga 520-0292 (JP)**

(74) Representative: **Vossius & Partner
Patentanwälte Rechtsanwälte mbB
Siebertstrasse 3
81675 München (DE)**

(54) **POROUS COMPOSITE BODY, BONE REGENERATION MATERIAL, AND METHOD FOR PRODUCING POROUS COMPOSITE BODY**

(57) An object of the present invention is to provide a porous composite that has adequate bending strength and excellent compressive strength and that contains OCP and collagen; and a bone regeneration material containing this porous composite. The present invention provides a porous composite containing octacalcium phosphate and collagen, and having a bending strength of 0.05 to 0.16 MPa and a compressive strength of 0.3 MPa or more.

**Description**

Technical Field

The present invention relates to a porous composite, a bone regeneration material, and a method for producing the porous composite.

Background Art

Calcium phosphates such as hydroxyapatite (HA) are known as conventionally used bone regeneration materials (see, for example, Patent Literature (PTL) 2, PTL 3, PTL 4, and PTL 5).
In recent years, octacalcium phosphate (hereinafter referred to as "OCP"), which is a precursor of HA, has been found to have higher bone regeneration-promoting action than other calcium phosphates, such as HA and β-tricalcium calcium phosphate (β-TCP), and also have high absorbability in living bodies (see, for example, Patent Literature (PTL) 1). Among calcium phosphates, OCP thus has particularly excellent properties as a bone regeneration material.
However, OCP, which is an inorganic substance, has poor shape-imparting properties. It is therefore difficult to apply OCP alone to regenerate an extensive bone defect region or the like. In light of this difficulty, use of a composite of OCP and collagen as a bone regeneration material has been proposed (see, for example, PTL 1).

Citation List

Patent Literature

PTL 1: JP2006-167445A
PTL 2: JP2010-273847A
PTL 3: JP2003-260124A
PTL 4: JP2009-132601A
PTL 5: JP2005-279078A
PTL 6: JPH5-070113A

Summary of Invention

Technical Problem

Bone regeneration materials are generally applied to indefinite bone defects. Bone regeneration materials used for this purpose are required to have excellent operability in clinical application, as well as the ability to promote bone regeneration. For example, characteristics such as (1) having an adequate bending strength, which allows easy processing in conformity with the shape of the bone defect site using, for example, tweezers, and (2) having an adequate strength and thus being less likely to be damaged etc. in handling are required.
Patent Literature (PTL) 4 discloses a porous body comprising apatite/collagen composite fibers, which has mechanical strength and bio-compatibility in an optimum balance, and which is suitable for use as artificial aggregates, scaffolds of cells, etc. Such apatite/collagen composite fibers are advantageous in terms of initial mechanical strength because they comprise fine apatite particles deposited on collagen; however, due to their strength, such apatite/collagen composite fibers have difficulty in easy processing using, for example, tweezers.
Patent Literature (PTL) 1 discloses a rigid tissue alternative carrier material comprising octacalcium phosphate and collagen, and having excellent shape-imparting properties. Such a rigid tissue alternative carrier material is highly flexible, but has a low compressive strength due to its large pore size, and thus has a problem of being easily damaged etc. during handling.
The present invention was made in view of the above problems. An object of the present invention is to provide a porous composite, containing OCP and collagen, that has improved bending strength and compressive strength, and good operability; a bone regeneration material containing the porous composite; and a method for producing the porous composite.

Solution to Problem

The present inventors conducted extensive research in view of the above problems. The inventors found that a porous composite that has a bending strength and a compressive strength within specific ranges, and that can thus achieve both easy processing and mechanical strength, is obtained by using a collagen-containing liquid having a specific ionic strength. As a result of further research and improvements based on this finding, the present inventors have accomplished the present invention. The present inventors provide inventions such as the following.

[1] A porous composite comprising octacalcium phosphate and collagen, and having a bending strength of 0.05 to 0.16 MPa and a compressive strength of 0.3 MPa or more.
[2] The porous composite according to [1], which has a pore size of 3 to 90 $\mu$m.
[3] The porous composite according to [1] or [2], wherein the collagen is type I collagen, or a mixture of type I collagen and type III collagen.
[4] A bone regeneration material comprising the porous composite according to any one of [1] to [3].
[5] A method for producing a porous composite comprising octacalcium phosphate and collagen, the method comprising the steps of:

(1) adjusting a collagen-containing liquid to an ionic strength of 0.005 to 0.06 and to a pH of 6.0 to 9.0 to prepare a collagen-containing gel, sol, or liquid; and
(2) mixing the collagen-containing gel, sol, or liquid with octacalcium phosphate to obtain a suspension.

[6] The method for producing a porous composite according to [5], further comprising the step of:

(3) immersing the suspension in a liquid refrigerant to quickly freeze the suspension, and then freeze-drying.

[7] A method for producing a porous composite comprising octacalcium phosphate and collagen, the method comprising the steps of:

(1) mixing a collagen-containing liquid with octacalcium phosphate to obtain a suspension; and
(2) adjusting the suspension to an ionic strength of 0.005 to 0.06 and to a pH of 6.0 to 9.0.

[8] The method for producing a porous composite according to [7], further comprising the step of:

(3) immersing the suspension in a liquid refrigerant to quickly freeze the suspension, and then freeze-drying.

Advantageous Effects of Invention

According to the present invention, there can be provided a porous composite comprising OCP and collagen and having a higher bending strength and higher compressive strength than conventional porous composites, and thus has excellent processability and is less likely to be damaged; and a bone regeneration material comprising the porous composite. Furthermore, the porous composite of the present invention is less susceptible to degradation by proteolytic enzymes, and can remain longer in the portion requiring bone regeneration; the porous composite is thus expected to promote bone regeneration.

Brief Description of Drawings

Fig. 1 is a schematic diagram for describing the size of bending test samples.
Fig. 2 is a schematic diagram for describing the method for measuring the bending strength.
Fig. 3 is a graph showing a stress-strain curve obtained by subjecting the composite of Example 1 to a bending strength test.
Fig. 4 is a schematic diagram for describing the method for measuring the compressive strength.

Description of Embodiments

Porous Composite

The porous composite of the present invention is a porous composite containing OCP and collagen (OCP/collagen composite). A preferred embodiment of the porous composite of the present invention is a porous composite in which collagen forms a three-dimensional, sponge-like structure, and OCP is present in the sponge-like structure as granules having a size of at least 100 $\mu$m or more. The bone regeneration material comprising the porous composite according to the present invention allows osteoblasts to enter inside the porous composite due to its porous structure, and promotes new bone formation, thus achieving internal bone formation.

OCP ($Ca_8H_2(PO_4)_6 \cdot 5H_2O$) can be prepared by various known methods. For example, OCP can be prepared by the drop method of LeGeros (LeGeros R.Z., Calcif Tissue Int 37: 194-197, 1985) or the method using a synthesizing apparatus (three-way pipe) disclosed in PTL 6. Alternatively, OCP can be obtained by a mixing method in which an aqueous sodium dihydrogen phosphate solution and an aqueous calcium acetate solution are mixed under appropriate conditions, and the generated precipitate is collected. The OCP obtained from the precipitate is preferably used after it is dried and ground into a granular powder using an electric mill or the like. The particle size is preferably in the range of 10 to 1000 $\mu$m, more preferably 100 to 500 $\mu$m, and even more preferably 300 to 500 $\mu$m. The particle size can be classified, for example, by sieving based on the opening size of the sieve.

The source, properties, etc., of the collagen are not particularly limited, and various collagens can be used. In one embodiment, the collagen is preferably an enzyme-solubilized collagen that is obtained by solubilizing collagen with protease (e.g., pepsin or pronase) and from which telopeptide is removed. The collagen is preferably type-I, type-II, type-III, or type-IV collagen, which is fibrous collagen. Type-I collagen, which is contained in a large amount in living bodies, or a mixture of type-I collagen and type-III collagen is particularly preferable. The raw material of collagen is not particularly limited. Collagen derived from skin, bone, tendon, or the like of pigs, cows, etc. can be preferably used. Collagen is an organism-derived component and is thus characterized by being highly safe. In particular, enzyme-solubilized collagen is preferable because of its low allergenicity. Commercially available collagen can also be used as the collagen.

The mixing ratio of OCP to collagen in the porous composite of the present invention can be appropriately adjusted according to the desired shape-imparting properties, operability, biocompatibility, etc. For example, the mixing ratio of calcium phosphate to 1 part by weight of collagen is 0.5 to 35 parts by weight, preferably 1 to 20 parts by weight, and more preferably 2 to 10 parts by weight. If the amount of calcium phosphate is less than 0.5 parts by weight based on 1 part by weight of collagen, the obtained composite may have an inferior bone regeneration function. If the amount of calcium phosphate is more than 35 parts by weight, inferior shape-imparting properties may result.

The porous composite of the present invention preferably has a bending strength of 0.05 to 0.16 MPa, and more preferably 0.08 to 0.16 MPa. When the bending strength exceeds 0.16 MPa, easy processing using tweezers or the like tends to be difficult. More specifically, when a doctor applies the porous composite of the present invention to a bone defect site, the size of the porous composite must be adjusted according to the size of the defect site. In this adjustment, when the bending strength is 0.16 MPa or less, the porous composite can be easily cut by applying adequate stress using tweezers. When the bending strength is less than 0.05 MPa, the porous composite is easily broken, and post-surgical breakage tends to occur.

The bending strength in the present invention is measured by the following method.

Bending Strength Measurement

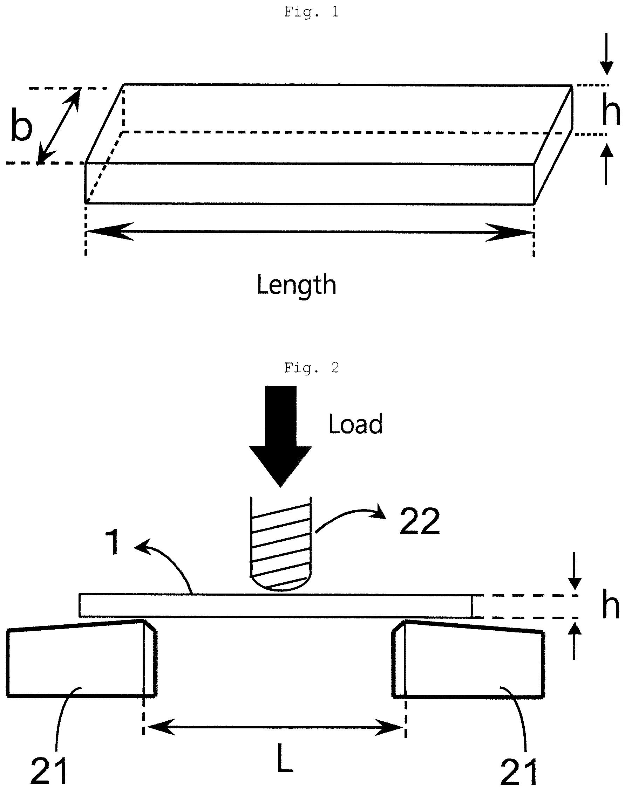

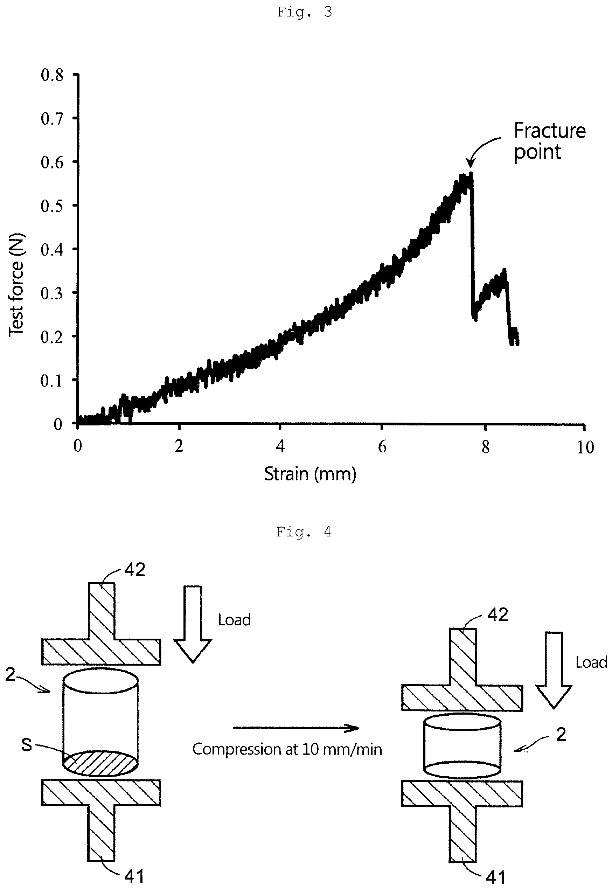

A rectangular parallelepiped specimen (sample) having a thickness (h) of 4 mm, a width (b) of 10 mm, and a length of 30 mm, shown in Fig. 1, is immersed in phosphate buffered saline (10 mM sodium phosphate, 0.14 M sodium chloride, pH 7.4) in a 25°C temperature, 65% humidity environment for 30 minutes. The water on the surface of the sample is then lightly blotted, and the sample is set on a support (support radius R1: 5 mm) of a tension and compression tester (load cell capacity: 1 kN) with a support span (L) of 20 mm. An indenter with a radius R2 of 5 mm is used as the indenter, which is a jig for applying a load to the sample. This measurement is performed by a 3-point bending assay, as shown in Fig. 2. Subsequently, the crosshead of the tension and compression tester is lowered at a rate of 10 mm/min until the sample breaks. The phrase "the sample broke" in this test means the point at which the load sharply decreases in the stress-strain curve, as shown in Fig. 3.

The bending strength ($\sigma$) in the present invention is calculated from the load at break of the sample, sample size, and support span according to Formula 1.

$$\sigma = 3FL/2bh^2 \quad (Formula\ 1)$$

σ: Bending strength (Pa)

F: Load at break (N)

L: Support span (mm)

b: Sample width (mm)

h: Sample thickness (mm)

The compressive strength of the porous composite of the present invention is preferably 0.3 MPa or more, more preferably 0.3 to 3.0 MPa, and even more preferably 0.3 to 1.0 MPa. When the compressive strength is less than 0.3 MPa, the operability of the porous composite tends to be reduced. In other words, when a bone defect site is filled with the porous composite of the present invention, collapse of the porous composite or crushing of pores, if occurred, would adversely affect the subsequent bone regeneration. Specifically, a compressive strength of 0.3 MPa or more is the index that indicates that neither collapse of the composite nor crushing of pores occurs when a doctor performs a procedure of filling bone defect sites having various shapes with the porous composite without leaving any clearance by using a jig. Although the upper limit of the compressive strength is not particularly specified, it is preferably 3.0 MPa or less in terms of ease of operation in filling a bone defect site.

The compressive strength in the present invention is measured by the following method.

Compressive Strength Measurement

A cylindrical specimen (sample) having a diameter of 8.5 mm and a height of 15 mm is immersed in a phosphate buffered saline (10 mM sodium phosphate, 0.14 M sodium chloride, pH 7.4) in a 25°C temperature, 65% humidity environment for 30 minutes. The water on the surface of the sample is then lightly blotted. A uniaxial load is applied in the vertical direction of the cylinder using a tension and compression tester (load cell capacity: 1 kN). The load is changed stepwise, and the smallest load at which the sample collapses is defined as the load at collapse. The phrase "collapse of the sample was observed" means that occurrence of a clear crack or peeling was confirmed when the sample was observed with the naked eye.

Specifically, with reference to Fig. 4, after a sample is set on a sample stage 41, a crosshead 42 to which a flat compression jig is attached is lowered at a rate of 10 mm/min. When the load cell indicates 2.5 N, the crosshead 42 is stopped to release the sample from the load. The sample on the sample stage is observed. If the sample is not collapsed, the sample is returned to the sample stage and set again, and the crosshead is lowered in the same manner until the load cell indicates 5 N. While the load is increased by 2.5 N every time, this operation is repeated. The load when collapse of the sample is first observed is defined as the load at collapse.

The compressive strength in the present invention can be calculated from the load at collapse, and the cross-sectional area of the sample (cross-sectional area in the section perpendicular to the thickness direction of the cylinder) according to Formula 2.

$$Cs = F/S \quad (Formula\ 2)$$

Cs: Compressive strength (Pa)

F: Load at collapse (N)

S: Cross-sectional area of sample (m$^2$)

The cross-sectional area of the sample is about $(0.00425)^2 \times 3.14 = 5.67 \times 10^{-5}$ m$^2$.

The porous composite of the present invention preferably has a pore size of 3 to 90 μm. If the pore size is 90 μm, the compressive strength of the porous composite tends to be reduced. On the other hand, if the pore size is less than 3 μm, it is difficult for bone metabolic cells, such as osteoblasts, to enter the porous composite, and the action of promoting bone regeneration may be reduced. The porous composite of the present invention more preferably has a pore size of 5 to 40 μm.

The pore size is measured by pore distribution measurement using a mercury porosimeter. The specific measurement method is as follows.

Measurement of Pore Size

As a pretreatment, samples (porous composites) are dried at a constant temperature of 120°C for 4 hours. Each of the pretreated samples is measured for pore distribution of the pore size of 0.0018 to 200 μm by a mercury penetration method using the following measuring apparatus under the following conditions: Measuring apparatus: AutoPore IV 9520 (produced by Micromeritics) Measurement condition: contact angle between mercury and sample:

140 deg

Surface tension of mercury: 0.48 N/m (in terms of 1 dyne = $10^{-5}$ N)

The "pore size" in this invention refers to the pore diameter value of the highest peak having the largest area in the pore distribution curve obtained from the measured pressure by the mercury penetration method.

The porosity (percentage of void) of the porous composite is preferably 80 to 99%, and more preferably 85 to 98%. The porosity is determined by the following formula from the total pore volume measured by the mercury penetration method and apparent density.

$$\texttt{Porosity (\%) = Total pore volume / \{(1/Apparent density) + Total}$$
$$\texttt{pore volume\} x 100}$$

The shape of the porous composite of the present invention is preferably a rectangular parallelepiped (block body), a cylinder, a tablet, or a granule. When the porous composite is a rectangular parallelepiped, the porous composite preferably has a size of greater than or equal to 5 mm x 5 mm x 5 mm. In general, the upper limit is preferably in the range of 100 mm x 100 mm x 100 mm. The rectangular parallelepiped is not limited to a cube. When the porous composite is cylindrical, the porous composite preferably has a diameter of 5 to 50 mm and a height in the range of 1 to 50 mm. When the porous composite is granular, the shape of the granule is not limited to spheres, and may be indefinite; however, the porous composite preferably has a diameter of 0.1 to 10 mm.

The porous composite of the present invention is used in such a manner that a bone defect site is filled with the porous composite. When sufficient blood or body fluid is present at the bone defect site, the bone defect site can be filled with the porous composite as is, or with the porous composite cut into an appropriate shape. When sufficient blood or the like is not present at the bone defect site, or when the porous composite cannot be used in its original form to fill the bone defect site therewith, the bone defect site can be filled with the porous composite after the porous composite is immersed in blood, physiological saline, or the like and spongy elasticity of the porous composite is confirmed.

Method for Producing Porous Composite

The method for producing the porous composite of the present invention is preferably a production method comprising mixing OCP with collagen. For example, the production methods described below can be used. If known coprecipitation methods or the like are used to deposit OCP on collagen and thereby form a composite of OCP and collagen, an excessively high bending strength may result, and easy processing may be difficult.

(a) Method for forming a composite by mixing OCP

First, a gelled collagen solution having a concentration of 0.1 to 5 wt.% and a pH of 6.0 to 9.0 is prepared and OCP is added thereto. The resulting mixture is kneaded to prepare a mixture of OCP and collagen. Subsequently, the mixture is molded in a suitable mold, then frozen, and freeze-dried to obtain a composite. If necessary, the obtained composite is subjected to heat dehydration crosslinking treatment, and further sterilized by a conventional sterilization method (e.g., $\gamma$-ray irradiation, electron beam irradiation, or ethylene oxide gas).

(b) Method for forming a composite by mixing an OCP suspension

A collagen acidic solution in a suitable concentration is aseptically adjusted to a pH of 5.5 to 7.5 using a suitable buffer (e.g., phosphate buffer, tris buffer, sodium acetate buffer, etc.). OCP is added thereto before the collagen is gelled, thereby preparing a suspension of collagen and OCP. The suspension is then poured into a mold while maintaining a neutral to weakly alkaline pH. After molding, the suspension is gelled at a suitable temperature (e.g., 37°C), and washing with water is repeated to remove salts of the buffer, etc. A composite can be thus obtained. The composite is preferably freeze-dried and sterilized in the same manner as described above.

The method for producing the porous composite of the present invention preferably comprises the step of adjusting the collagen-containing liquid to an ionic strength of 0.005 to 0.06 and a pH of 6.0 to 9.0 to prepare a collagen-containing gel, sol, or liquid. The porous composite more preferably has an ionic strength of 0.005 to 0.05, and a pH of 6.5 to 8.0. In the method (a), this step is performed before adding OCP. In the method (b), this step is performed after adding OCP and preparing an OCP/collagen suspension. The ionic strength and pH can be adjusted by adding a commonly used buffer and salt to a solution of collagen in distilled water. Examples of buffers include acetate buffer, phosphate buffer, tris buffer, and Good's buffers such as MES buffer and HEPES buffer. Examples of salts include sodium chloride (NaCl), potassium chloride, sodium hydroxide, dilute hydrochloric acid, and the like.

In the present invention, ionic strength ($\mu$) is calculated according to Formula 3.

$$\mu = 1/2\Sigma\ (c_i z_i{}^2)\ \ (\text{Formula 3})$$

$\mu$: Ionic strength

$c_i$: Molar concentration of ion

$z_i$: Electric charge of ion

For example, the electrolyte present in Example 1 described below is only 0.01M NaCl, which is the total of NaCl contained in collagen and NaCl generated by pH adjustment. Thus, the ionic strength is $1/2 \times (0.01 \times 1^2 + 0.01 \times 1^2) = 0.01$.

In general, collagen is known to efficiently self-assemble (fibrose) under physiological conditions (ionic strength: about 0.2, pH: about 7.0). The present inventors found that adjusting the ionic strength and pH of the collagen-containing liquid to the specific ranges of the present invention is crucial in determining the properties of the porous composite, such as compressive strength and bending strength. It is considered that in the present invention, collagen is allowed to fibrose under conditions lower than physiological ionic strength, whereby the collagen fiber diameter, fiber length, and interaction between collagen molecules can be appropriately controlled, and a preferable bending strength can be obtained.

The method for producing the porous composite of the present invention preferably comprises the step of immersing an OCP- and collagen-containing gel, sol, or liquid in a liquid refrigerant to quickly freeze the gel, sol, or liquid and then freeze-drying. The liquid refrigerant is a liquid having a temperature lower than the freezing temperature of the OCP- and collagen-containing gel, sol, or liquid. Examples of liquids include methanol, ethanol, acetone, acetonitrile, and liquid nitrogen. The temperature of the liquid refrigerant is preferably -20°C or less, more preferably -40°C or less, and still more preferably -80°C or less.

It is considered that the pore size of the obtained porous composite can be reduced by quickly freezing an octacalcium phosphate- and collagen-containing gel, sol, or liquid by immersion in a liquid refrigerant.

The porous composite of the present invention is preferably subjected to a heat treatment or dehydrothermal crosslinking treatment. As a result of the heat treatment, a part of the OCP molecular structure collapses to facilitate entry of osteogenic cells and promote bone regeneration, and also cause collagen to be cross-linked, thus improving shape retention.

The temperature of the heat treatment is preferably 50 to 200°C, and more preferably 60 to 180°C. The heat treatment is preferably performed under reduced pressure. The pressure is preferably 0 to 3000 Pa, and more preferably 0 to 300 Pa. The treatment time of the heat treatment is preferably 2 hours to 10 days, and more preferably 12 hours to 5 days.

Another effect of the porous composite produced by the above method is excellent control of the biodegradation rate. The present inventors found that biodegradability can be controlled by ionic strength of the collagen suspension in the production of a porous composite. Since bone regeneration takes a period of several weeks to several months, it is preferable for the porous composite to be present at a bone defect site for a long period of time and remain to function as a scaffold for bone regeneration. The production method of the present invention can provide a less biodegradable porous composite. The degree of degradability can be evaluated by the proportion of solubilized proteins. More specifically, the porous composite is immersed in a 0.2 mg/ml collagenase solution and allowed to stand at 37°C for 72 hours. The solubilized proteins of collagen present in the solution were subjected to BCA (Bicinchoninic Acid) protein assay to measure the protein amount. The solubilized protein amount is divided by the original collagen weight of the porous composite to determine the solubilized protein proportion (%). A preferable proportion of solubilized proteins in the porous composite of the present invention is 1.5% to 4.0%.

Bone Regeneration Material

The present invention further relates to a bone regeneration material containing the porous composite. The bone regeneration material is useful, for example, for bone defect repair in the dental oral surgery field and orthopedic surgery field, and for bone defect repair after craniotomy or thoracotomy. For example, in the dental oral surgery field, when a bone defect caused by periodontal disease, a cystic cavity, an atrophic alveolar ridge, a cleft jaw, a tooth-extraction cavity, or the like is filled with a bone regeneration material comprising the porous composite, an excellent bone regeneration effect can be confirmed after several weeks to several months. In the orthopedic surgery field, for example, in the case of a bone defect after resection of a bone tumor or a bone defect caused by trauma, such as a bone fracture, the bone defect site can be filled with the bone regeneration material of the present invention to thereby promote bone regeneration.

The bone regeneration material may contain, in addition to OCP and collagen, for example, cytokine (e.g.,

bone morphogenetic protein-2, transforming growth factor $\beta 1$, etc.) having a bone-forming ability. Containing such cytokine can increase the bone regeneration speed.

The bone regeneration material may further contain other ingredients that are commonly used in this field. Examples of such ingredients include bioabsorbable polymers (e.g., polyglycolic acid, polylactic acid, polylactic acid-polyethylene glycol copolymer etc.), and bioabsorbable calcium phosphates other than OCP (e.g., $\beta$-TCP).

Examples

The present invention is described in more detail below with reference to Examples; however, the present invention is not limited thereto.

Example 1

(1) Preparation of OCP

Liquid 1 and liquid 2 for preparation of OCP were prepared in the following manner.

Liquid 1: 31.2 g of sodium dihydrogen phosphate dihydrate was dissolved in 2500 g of distilled water to prepare liquid 1.
Liquid 2: 35.2 g of calcium acetate monohydrate was dissolved in 2500 g of distilled water to prepare liquid 2.

Subsequently, liquid 1 was placed in a separable flask and heated to 70°C using a mantle heater. While stirring liquid 1 at a rate of 250 rpm using a stirrer (MAZELA Z, produced by Tokyo Rikakikai Co., Ltd.) to which a stirring blade (blade diameter: 12 cm) was attached, liquid 2 was added dropwise to liquid 1 at a rate of about 28 mL/min. After completion of the dropwise addition, the mixture of liquid 1 and liquid 2 was further stirred at 70°C at 250 rpm for 2 hours.

The precipitate produced in the above mixture was then filtered through a membrane filter (pore size: 3 $\mu$m; A300A293C, produced by Advantec Toyo Kaisha, Ltd.) and collected. The collected precipitate was dispersed in 1500 mL of distilled water, and washed by stirring for 15 minutes. The process of filtering and washing was further repeated three more times.

Subsequently, the washed precipitate was dried in a constant-temperature dryer at 30°C for 24 hours. After the dried precipitate was ground with an electric mill, the ground product was screened through sieves to obtain particles with a particle size of 300 to 500 $\mu$m. A powder was thus obtained. Finally, the obtained powder was subjected to dry-heat sterilization at 120°C for 2 hours.

(2) Preparation of OCP/Collagen Composite (porous composite)

One part by weight of pig dermis-derived collagen containing type-I collagen and type-III collagen (NMP Collagen PS, produced by NH Foods Ltd.) was dissolved in 200 parts by weight of 4°C cooled distilled water to obtain an about 0.5 wt.% collagen solution. While maintaining the solution temperature at 4°C, an aqueous sodium hydroxide solution was added to the aqueous collagen solution to adjust the pH to about 7.4, thus obtaining a collagen suspension. The ionic strength of the collagen suspension at this time was about 0.01. The OCP (particle size: 300 to 500 $\mu$m) obtained in Production Example 1 was added to the collagen suspension so that the weight ratio of OCP to collagen was 77:23. The mixture was stirred at room temperature, thereby obtaining an OCP/collagen suspension.

Subsequently, the OCP/collagen suspension was placed in a centrifuge bottle, and centrifuged by a centrifugal separator (GRX-250, produced by Tomy Seiko Co., Ltd.) at a centrifugal force of 7000 x g for 20 minutes. The supernatant was then discarded so that the amount of collagen in the OCP/collagen suspension was 3 wt.% to obtain an OCP/collagen composite gel. The gel was placed in a plastic container having a cylindrical inner space (inner diameter: 8.5 mm, volume: about 3.0 cm$^3$) or a plastic vessel having a rectangular parallelepiped inner space (10 mm x 10 mm x 50 mm), and centrifuged with a centrifugal force of 230 x g for 1 minute to remove bubbles.

Each of the containers was sealed and immersed in a large excess volume of -80°C cooled methanol relative to the volume of the object to be frozen so as to quickly freeze the composite gel. After the containers were opened, the frozen products were dried with a freeze-dryer (-10°C, 48 hours) to form the products. Subsequently, the formed products were heated at 150°C for 24 hours at reduced pressure to perform thermal dehydration crosslinking. Using a scalpel, the cylindrical product was cut to a thickness of 15 mm, and the rectangular parallelepiped product was cut to a size of 4 mm x 10 mm x 30 mm. Finally, the cut pieces were sterilized by electron beam irradiation (15 kGy). The porous composites (OCP/collagen composites) of Example 1 were thus obtained.

Example 2

In Example 2, porous composites (OCP/collagen composites) were produced in the same manner as in Example 1, except that type-I collagen- and type-III collagen-containing pig dermis-derived collagen with a low NaCl content was used to prepare porous composites. The low-salt collagen was prepared in the following manner.

Preparation of low-salt collagen

One part by weight of pig dermis-derived collagen containing type-I collagen and type-III collagen (NMP Collagen PS, produced by NH Foods Ltd.) was dissolved in 200 parts by weight of 4°C cooled distilled water to obtain an about 0.5 wt.% collagen solution. The amount of NaCl contained in the pig dermis-derived collagen was 4%. While maintaining the solution temperature at 4°C, an aqueous sodium hydroxide solution was added to the aqueous collagen solution to adjust the pH to about 8.0, thus obtaining a collagen suspension. The collagen suspension was placed in a centrifuge bottle, and centrifuged by a centrifugal separator (GRX-250, produced by Tomy Seiko Co., Ltd.) at a centrifugal force of 7000 x g for 20 minutes. The collagen gel obtained by completely discarding the supernatant was frozen in a freezer at -35°C. The frozen product was dried using a freeze-dryer to obtain a low-salt collagen. The amount of NaCl contained in the low-salt collagen was measured by atomic absorption spectrophotometry (ashing), and found to be 1 wt.%.

Example 3

In Example 3, porous composites (OCP/collagen composites) were produced in the same manner as in Example 1, except that phosphate buffered saline (PBS) was added to the collagen suspension of Example 1 to adjust the ionic strength to 0.05.

Comparative Example 1

One part by weight of pig dermis-derived collagen containing type-I collagen and type-III collagen (NMP Collagen PS, produced by NH Foods Ltd.) was dissolved in 200 parts by weight of 4°C cooled distilled water to obtain an about 0.5 wt.% aqueous collagen solution. While maintaining the liquid temperature at 4°C, an aqueous sodium hydroxide solution was added to the aqueous collagen solution to adjust the pH to about 7.4, thus obtaining a collagen suspension. Phosphate buffered saline (PBS) was added to the collagen suspension to adjust the ionic strength to 0.1. Subsequently, the OCP (particle size: 300 to 500 $\mu$m) obtained in Example 1 was added to the collagen suspension so that the weight ratio of OCP to collagen was 77:23. The mixture was further stirred at room temperature to obtain an OCP/collagen suspension.

Subsequently, the obtained OCP/collagen suspension was placed in a centrifuge bottle, and centrifuged by a centrifugal separator (GRX-250, produced by Tomy Seiko Co., Ltd.) at a centrifugal force of 7000 x g for 20 minutes. The supernatant was then discarded so that the amount of collagen in the OCP/collagen suspension was 3 wt.%, thus obtaining an OCP/collagen composite gel. The composite gel was placed in a plastic container having a cylindrical inner space (inner diameter: 8.5 mm, volume: about 3.0 cm$^3$) or in a plastic container having a rectangular parallelepiped inner space (10 mm x 10 mm x 50 mm), and centrifuged at a centrifugal force of 230 x g for 1 minute to remove bubbles.

Each of the containers was sealed and then immersed in a large excess volume of -80°C cooled methanol relative to the volume of the object to be frozen, so as to quickly freeze the composite gel. After the containers were opened, the frozen products were dried by a freeze-dryer (-10°C, 48 hours) to form the products. Subsequently, the formed products were heated at 150°C for 24 hours at reduced pressure to perform thermal dehydration crosslinking. Using a scalpel, the cylindrical product was cut to a thickness of 15 mm, and the rectangular parallelepiped product was cut to a size of 4 mm x 10 mm x 30 mm. Finally, the cut pieces were sterilized by electron beam irradiation (15 kGy). The porous composites (OCP/collagen composites) of Comparative Example 1 were thus obtained.

Comparative Example 2

In Comparative Example 2, porous composites (COP/collagen composites) were obtained in the same manner as in Comparative Example 1, except that phosphate buffered saline (PBS) was added to the collagen suspension of Comparative Example 1 to adjust the ionic strength to 0.15.

Comparative Example 3

In Comparative Example 3, porous composites (OCP/collagen composites) were prepared in the same manner as in Comparative Example 1, except that: while maintaining the liquid temperature at 4°C, an aqueous sodium hydroxide solution was added to the about 0.5 wt.% aqueous collagen solution to obtain a collagen suspension having a pH of about 7.4 and an ionic strength of 0.01; and each container containing the OCP/collagen composite gel was placed in a freezer at -80°C to freeze the composite in place of immersing the container in -80°C cooled methanol to quickly freeze the composite.

Comparative Example 4

Preparation of hydroxyapatite/collagen composite

Pig dermis-derived collagen containing type I and type III collagens (NMP collagen PS, produced by NH Foods Ltd.) was dissolved in 4°C cooled distilled water to obtain an about 0.8 wt.% collagen solution. An equal volume of a mixture of a disodium hydrogen phosphate solution and a sodium chloride solution (30 mM $Na_2HPO_4$, 70 mM NaCl) was added to this collagen solution, and the resulting mixture was stirred to obtain a collagen suspension. The ionic strength of the collagen suspension at this time was about 0.07.

The collagen suspension was homogenized, then placed in a centrifuge bottle, and centrifuged by a centrifugal separator (GRX-250, produced by Tomy Seiko Co., Ltd.) at a centrifugal force of 20000 x g for 20 minutes. Subsequently, the supernatant was discarded so that the amount of collagen in the collagen suspension was 5 wt.% to obtain a 5 wt.% collagen gel.

Hydroxyapatite (HA) (Apatite HAP, monoclinic, produced by Wako Pure Chemical Industries, Ltd.) was added to the 5 wt.% collagen gel so that the weight ratio of HA to collagen was 60:40, and the resulting mixture was then mixed with a spatula. The mixture was placed in a plastic container having a cylindrical inner space (inner diameter: 8.5 mm, capacity: about 3.0 cm$^3$) or in a plastic container having a rectangular parallelepiped inner space (10 mm x 10 mm x 50 mm), and centrifuged with a centrifugal force of 230 x g for 1 minute to remove bubbles.

Each of the containers was hermetically sealed, and placed in a freezer at -20°C to freeze the mixture. After the containers were opened, the frozen products were dried by a freeze-dryer (-10°C, 48 hours) to form the products. Then, the formed products were heated at 150°C for 24 hours under reduced pressure to perform dehydrothermal crosslinking. Using a scalpel, the cylindrical product was cut to a thickness of 15 mm and the rectangular parallelepiped product was cut to a size of 4 mm x 10 mm x 30 mm. Finally, the cut pieces were sterilized by electron beam irradiation (15 kGy). The HA/collagen composites of Comparative Example 4 were thus obtained.

Comparative Example 5

In Comparative Example 5, HA/collagen composites of Comparative Example 5 were produced in the same manner as in Comparative Example 4, except that: after 5 wt.% collagen gel was obtained by centrifugal concentration, the gel was homogenized again and centrifuged at 20000 x g for 20 minutes in the same manner as above; finally, the supernatant was removed so that the amount of collagen in the collagen suspension was 10 wt.%, thus obtaining 10 wt.% collagen gel; and hydroxyapatite (HA) (Apatite HAP, monoclinic, produced by Wako Pure Chemical Industries, Ltd.) was added to the 10 wt.% collagen gel so that the weight ratio of HA to collagen was 20:80, and the resulting mixture was then mixed with a spatula.

Three samples for each of Examples 1 to 3 and Comparative Examples 1 to 5 were prepared. The bending strength and compressive strength of each sample were measured, and the averages were calculated. Specifically, the following methods were used to measure the bending strength and compressive strength in the Examples.

Bending Strength Measurement

A rectangular parallelepiped specimen having a thickness (h) of 4 mm, a width (b) of 10 mm, and a length of 30 mm, as shown in Fig. 1, was immersed in phosphate buffered saline (10 mM sodium phosphate, 0.14 M sodium chloride, pH 7.4) in a 25°C temperature, 65% humidity environment for 30 minutes. The water on the surface of the sample was then lightly blotted. As shown in Fig. 2, a load was applied to the sample using a precision universal testing machine (Autograph AGS-J, produced by Shimadzu Corporation, load cell capacity: 1 kN). The bending strength was determined from the obtained stress-strain curve.

Specifically, with reference to Fig. 2, after a sample 1 was set to a support 21 (radius of the support R1: 5 mm), the crosshead 22 to which an indentor with a radius R2 of 5 mm was attached was lowered at a rate of 10 mm/min until the sample broke. The phrase "the sample broke" as used herein means the point at which the load sharply decreases in the stress-strain curve, as shown in Fig. 3.

The bending strength (σ) in the present invention is calculated from the load at break of the sample, sample size, and support span according to Formula 4.

$$\sigma = 3FL/2bh^2 \text{ (Formula 4)}$$

$\sigma$: Bending strength (Pa)
F: Load to fracture (N)
L: Support span (mm)
b: Sample width (mm)
h: Sample thickness (mm)

The support span in this test was 20 mm.

Compressive Strength Measurement

A cylindrical specimen having a diameter of 8.5 mm and a length of 15 mm was immersed in phosphate buffered saline (10 mM sodium phosphate, 0.14 M sodium chloride, pH 7.4) in a 25°C temperature, 65% humidity environment for 30 minutes. The water on the surface of the sample was then lightly blotted. As shown in Fig. 4, a uniaxial load was applied to the sample using a precision universal testing machine (Autograph AGS-J, produced by Shimadzu Corporation, load cell capacity: 1 kN). The load was changed stepwise, and the minimum load at which the sample collapsed was defined as a load at collapse.

Specifically, with reference to Fig. 4, after a sample 2 was set on a sample stage 41, a crosshead 42 to which a flat compression jig was attached was lowered at a rate of 10 mm/min. When the load cell indicated 2.5 N, the crosshead 42 was stopped to release the sample from the load. The sample on the sample stage was observed. If the sample was not collapsed, the sample was returned to the sample stage and set again. The crosshead was lowered in the same manner until the load cell indicated 5 N. This operation was repeated while the load was increased by 2.5 N every time. The load when collapse of the sample was first observed was defined as a load at collapse. The phrase "collapse of the sample was observed" in this test means that a clear crack or peeling was confirmed when the specimen was observed with the naked eye.

The compressive strength in the present invention can be calculated from the load at collapse, and the cross-sectional area of the sample (cross-sectional area in the section perpendicular to the thickness direction of the cylinder) according to Formula 5.

$$Cs = F/S \text{ (Formula 5)}$$

Cs: Compressive strength (Pa)
F: Load at collapse (N)
S: Cross-sectional area of sample ($m^2$)

The cross-sectional area of the sample was about $(0.00425)^2 \times 3.14 = 5.67 \times 10^{-5}$ $m^2$.

Table 1 shows measurement results of the bending strength and compressive strength of the composites obtained in the Examples and Comparative Examples.

Table 1

| | Freezing rate | Ionic strength | Composition of composite | Compressive strength (MPa) | Bending strength (MPa) |
|---|---|---|---|---|---|
| Example 1 | -80°C MeOH | 0.01 | OCP/Col 77:23 | 0.530 | 0.080 |
| Example 2 | -80°C MeOH | 0.005 | OCP/Col 77:23 | 0.516 | 0.058 |
| Example 3 | -80°C MeOH | 0.05 | OCP/Col 77:23 | 0.530 | 0.156 |
| Comparative Example 1 | -80°C MeOH | 0.1 | OCP/Col 77:23 | 0.543 | 0.223 |
| Comparative Example 2 | -80°C MeOH | 0.15 | OCP/Col 77:23 | 0.543 | 0.332 |

(continued)

|  | Freezing rate | Ionic strength | Composition of composite | Compressive strength (MPa) | Bending strength (MPa) |
|---|---|---|---|---|---|
| Comparative Example 3 | -80°C freezer | 0.01 | OCP/Col 77:23 | 0.250 | 0.053 |
| Comparative Example 4 | -20°C freezer | 0.07 | HA/Col 60:40 | 0.260 | 0.334 |
| Comparative Example 5 | -20°C freezer | 0.07 | HA/Col 20:80 | 0.233 | 0.266 |

The results in Table 1 show that the samples of Examples 1 to 3 (porous composites) had a bending strength of 0.05 to 0.16 MPa and a compressive strength of 0.3 MPa or more, and are excellent in terms of processability and ease of handling, compared with conventional porous composites.

The samples of Comparative Examples 1 and 2 had a compressive strength of 0.3 MPa or more, and were sufficient in terms of ease of handling; however, these samples had a bending strength of more than 0.16 MPa due to their high ionic strength at the time of collagen fibrosis, and easy processing was difficult.

The sample of Comparative Example 3 had an adequate bending strength, and can achieve ease of processing; however, since slow gas-phase cooling by a freezer was used to freeze the composite gel of OCP and collagen, the sample had a compressive strength of less than 0.3 MPa, resulting in poor handleability.

The samples of Comparative Examples 4 and 5 had a bending strength of more than 0.16 MPa due to their high ionic strength at the time of collagen fibrosis, and had a compressive strength of less than 0.3 MPa because slow gas-phase cooling by a freezer was used to freeze the composite gel of OCP and collagen. Therefore, these samples were deficient in processability and handleability.

Measurement of Degradability

The cylindrical samples of Example 1, Example 3, Comparative Example 1, and Comparative Example 2 were cut to a thickness of about 10 mm and immersed in a collagenase solution. The proportion of solubilized proteins of collagen was measured to determine the biodegradability of the porous composite.

Specifically, the cylindrical products obtained in Example 1, Example 3, Comparative Example 1, and Comparative Example 2 were cut to a thickness of about 10 mm and immersed in a 0.2 mg/mL collagenase type I solution (produced by Wako Pure Chemical Industries, Ltd., 180 Unit/mg), and allowed to stand at 37°C for 72 hours. As a control, a collagenase solution alone in which no samples were immersed was allowed to stand at 37°C for 72 hours. To measure the amount of solubilized proteins in the solution, absorbance at 570 nm was then measured using a BCA protein assay kit (Pierce BCA Protein Assay Kit, produced by Thermo Scientific) by a microplate reader (produced by BIO-RAD, iMark) to quantify the protein content of the supernatant ($\mu$g). Bovine serum albumin was used as a standard substance. The quantified protein content of the supernatant was divided by the collagen weight (mg) of each sample to determine the solubilized protein proportion (%). The collagen weight was calculated by multiplying the weight of the cylindrical product having a thickness of about 10 mm by 0.23. Table 2 shows the results.

Table 2

|  | Ionic strength | Collagen weight (mg) | Absorbance at 570 nm | Protein content of the supernatant ($\mu$g) | Solubilized protein proportion (%) |
|---|---|---|---|---|---|
| Example 1 | 0.01 | 16.18 | 0.538 | 266.25 | 1.65 |
| Example 3 | 0.05 | 11.98 | 0.706 | 423.75 | 3.54 |
| Comparative Example 1 | 0.10 | 15.55 | 0.978 | 678.75 | 4.36 |
| Comparative Example 2 | 0.15 | 18.00 | 1.393 | 1067.81 | 5.93 |
| Blank (only collagenase) | - | 0 | 0.254 | 186.66 | - |

The results in Table 2 show that there is a positive correlation between the ionic strength of the collagen suspension and the proportion of solubilized proteins; and that the sample having a higher ionic strength tends to be easily degraded by collagenase. Suppressing the degradability of the porous composite is expected to allow the porous composite to remain longer at a bone deficient site and function as a scaffold for bone regeneration, thus providing the effect of more effectively promoting bone generation.

It should be understood that the embodiments and examples disclosed herein are illustrative and non-restrictive in every respect. The scope of the present invention is defined by the claims, rather than the description above, and is intended to include any modifications within the scope and meaning equivalent to the claims.

Industrial Applicability

The porous composite and the bone regeneration material containing the porous composite of the present invention have a good balance of ease of processing and mechanical strength, and have excellent operability, as well as high bone regeneration ability. Therefore, the porous composite and the bone regeneration material are mainly useful for bone defect repair in the dental oral surgery field and orthopedic surgery field.

Description of Reference Numerals

1: Bending test sample

2: Compression test sample

21: Support

22: Indenter

41: Sample Stage

42: Crosshead

**Claims**

1. A porous composite comprising octacalcium phosphate and collagen, and having a bending strength of 0.05 to 0.16 MPa and a compressive strength of 0.3 MPa or more.

2. The porous composite according to claim 1, which has a pore size of 3 to 90 $\mu$m.

3. The porous composite according to claim 1 or 2, wherein the collagen is type I collagen, or a mixture of type I collagen and type III collagen.

4. A bone regeneration material comprising the porous composite according to any one of claims 1 to 3.

5. A method for producing a porous composite comprising octacalcium phosphate and collagen, the method comprising the steps of:

   (1) adjusting a collagen-containing liquid to an ionic strength of 0.005 to 0.06 and to a pH of 6.0 to 9.0 to prepare a collagen-containing gel, sol, or liquid; and
   (2) mixing the collagen-containing gel, sol, or liquid with octacalcium phosphate to obtain a suspension.

6. The method for producing a porous composite according to claim 5, further comprising the step of:

   (3) immersing the suspension in a liquid refrigerant to quickly freeze the suspension, and then freeze-drying.

7. A method for producing a porous composite comprising octacalcium phosphate and collagen, the method comprising the steps of:

(1) mixing a collagen-containing liquid with octacalcium phosphate to obtain a suspension; and

(2) adjusting the suspension to an ionic strength of 0.005 to 0.06 and to a pH of 6.0 to 9.0.

8. The method for producing a porous composite according to claim 7, further comprising the step of:

(3) immersing the suspension in a liquid refrigerant to quickly freeze the suspension, and then freeze-drying.

Fig. 1

Length

Fig. 2

Load

22

1

h

L

21                    21

Fig. 3

Fig. 4

## INTERNATIONAL SEARCH REPORT

| International application No. |
| --- |
| PCT/JP2016/061260 |

| A. CLASSIFICATION OF SUBJECT MATTER |
| --- |
| *A61L27/00(2006.01)i* |

According to International Patent Classification (IPC) or to both national classification and IPC

| B. FIELDS SEARCHED |
| --- |
| Minimum documentation searched (classification system followed by classification symbols)<br>A61L27/00 |

Documentation searched other than minimum documentation to the extent that such documents are included in the fields searched

| | | | |
| --- | --- | --- | --- |
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)
JSTPlus/JMEDPlus/JST7580(JDreamIII), CAplus/MEDLINE/EMBASE/BIOSIS(STN)

| C. DOCUMENTS CONSIDERED TO BE RELEVANT | | |
| --- | --- | --- |
| Category* | Citation of document, with indication, where appropriate, of the relevant passages | Relevant to claim No. |
| X | JP 06-304242 A (Nitta Gelatin Inc.),<br>01 November 1994 (01.11.1994),<br>entire text; particularly, paragraphs [0019],<br>[0023], [0024], [0026], [0031] to [0033]<br>(Family: none) | 1–4 |

| ☒ Further documents are listed in the continuation of Box C. | ☐ See patent family annex. |
| --- | --- |

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>06 June 2016 (06.06.16) | Date of mailing of the international search report<br>21 June 2016 (21.06.16) |
| --- | --- |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

International application No.

PCT/JP2016/061260

C (Continuation).   DOCUMENTS CONSIDERED TO BE RELEVANT

| Category* | Citation of document, with indication, where appropriate, of the relevant passages | Relevant to claim No. |
|---|---|---|
| X | Ken TAKAHASHI, "The Effect of Collagen Composed with Biodegradable Calcium Phosphate for Bone Substitute", The journal of the Tokyo Dental College Society, 1997, vol.97, no.5, pages 509 to 536, ISSN:0037-3710 page 510, left column, 3rd line from the bottom to page 510, right column, line 2, page 511, right column, 4th line from the bottom to page 512, left column, line 1, page 529, left column, 1st line from the bottom to page 529, right column, line 2, page 532, right column, line 15 to page 533, left column, line 22, page 533, right column, 7th to 1st lines from the bottom, table 1, fig. 1, 5 | 1-4 |
| X | KAWAI T. et al., First clinical application of octacalcium phosphate collagen composite in human bone defect., Tissue Engineering. Part A, 2014, Vol.20, No.7-8, pages 1336 to 1341, ISSN: 1937-3341 page 1337, left column, lines 36 to 55, page 1338, right column, lines 2 to 5, page 1336, right column, line 12 to page 1337, left column, line 10, page 1340, left column, line 7 to page 1340, right column, 4th line from the bottom, Fig. 1, Fig. 3cd | 1-4 |
| Y | JP 2006-167445 A  (Nippon Meat Packers, Inc.), 29 June 2006 (29.06.2006), entire text; particularly, paragraphs [0011], [0014], [0017], [0018], [0023] to [0027] (Family: none) | 5-8 |
| Y | WO 2013/005778 A1  (Tokyo Institute of Technology), 10 January 2013 (10.01.2013), entire text; particularly, paragraphs [0014], [0017], [0053], [0054], [0056] to [0060] & US 9119903 B2 column 6, lines 17 to 26; column 7, lines 12 to 27; column 14, line 58 to column 15, line 11; column 16, lines 14 to 41; column 17, lines 4 to 43 & EP 2730295 A1 | 5-8 |
| Y | WO 2012/070680 A1  (Tokyo Institute of Technology), 31 May 2012 (31.05.2012), paragraphs [0007], [0016] & US 2013/0337227 A1 paragraphs [0010], [0040] & EP 2644620 A1 | 5-8 |

Form PCT/ISA/210 (continuation of second sheet) (January 2015)

**INTERNATIONAL SEARCH REPORT**

International application No.

PCT/JP2016/061260

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2014-166217 A (Tokyo Institute of Technology), 11 September 2014 (11.09.2014), paragraphs [0009], [0034] (Family: none) | 5-8 |
| Y | JP 2010-273847 A (Tokyo Institute of Technology), 09 December 2010 (09.12.2010), paragraphs [0007], [0008], [0026], [0029], [0030] (Family: none) | 5-8 |
| Y | WO 2004/103422 A1 (Pentax Corp.), 02 December 2004 (02.12.2004), paragraphs [0031], [0049], [0052], [0061] & US 2006/0292350 A1 paragraphs [0035], [0053], [0056], [0065] & EP 1642599 A1 | 6,8 |
| Y | JP 2008-531230 A (Cambridge Enterprise Ltd.), 14 August 2008 (14.08.2008), paragraphs [0023], [0025], [0030], [0031], [0057] & US 2009/0022771 A1 paragraphs [0022], [0024], [0029], [0030], [0056] & EP 1855734 A1 | 6,8 |
| Y | WO 2014/126196 A1 (Osaka University), 21 August 2014 (21.08.2014), paragraph [0019] & US 2016/0089474 A1 paragraph [0044] | 6,8 |
| Y | Fumio MUTO, "Liquid Nitrogen Quick Freezing of Foods", Shinku, 1969, vol.12, no.5, pages 173 to 179, ISSN:0559-8516 page 174, right column, 8th to 5th lines from the bottom | 6,8 |

Form PCT/ISA/210 (continuation of second sheet) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006167445 A
- JP 2010273847 A
- JP 2003260124 A

- JP 2009132601 A
- JP 2005279078 A
- JP H5070113 A

**Non-patent literature cited in the description**

- **LEGEROS R.Z.** *Calcif Tissue Int,* 1985, vol. 37, 194-197